(19) **Europäisches Patentamt European Patent Office Office européen des brevets**

(11) **EP 2 978 499 B1**

(12) **EUROPEAN PATENT SPECIFICATION**

(45) Date of publication and mention
of the grant of the patent:
**30.01.2019 Bulletin 2019/05**

(51) Int Cl.:
*A61Q 1/06* (2006.01)  *A61K 8/25* (2006.01)
*A61K 8/37* (2006.01)  *A61K 8/02* (2006.01)

(21) Application number: **13880325.9**

(22) Date of filing: **25.03.2013**

(86) International application number:
**PCT/CN2013/073110**

(87) International publication number:
**WO 2014/153699 (02.10.2014 Gazette 2014/40)**

(54) **SOLID ANHYDROUS COMPOSITION COMPRISING A HIGH MELTING POINT WAX AND SILICA AEROGEL**

FESTE ANHYDRISCHE ZUSAMMENSETZUNG MIT EINEM WACHS MIT HOHEM SCHMELZPUNKT UND SILICA-AEROGEL

COMPOSITION ANHYDRE SOLIDES COMPRENANT UNE CIRE À POINT DE FUSION ÉLEVÉ ET UN AÉROGEL DE SILICE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**03.02.2016 Bulletin 2016/05**

(73) Proprietor: **L'Oréal**
**75008 Paris (FR)**

(72) Inventors:
• **XU, Zhen**
**Shanghai 200000 (CN)**
• **HUANG, Manlian**
**Shanghai 200000 (CN)**

(74) Representative: **Casalonga**
**Casalonga & Partners**
**Bayerstraße 71/73**
**80335 München (DE)**

(56) References cited:
WO-A1-02/058643  WO-A2-2012/084522
WO-A2-2012/085855  FR-A1- 2 968 979
US-A1- 2004 037 859  US-A1- 2008 171 005
US-A1- 2009 104 294  US-A1- 2011 052 522
US-A1- 2012 107 254

## Description

TECHNICAL FIELD

The present invention relates, in general, to a solid, anhydrous composition for application onto a targeted substrate. More particularly, the present invention relates to a non-liquid, anhydrous composition which has good pay-off, good stability, and shiny effect, particularly with respect to variations in temperature.

BACKGROUND OF THE INVENTION

Compositions for caring for and/or making up the skin and/or the lips generally contain one or more fatty substances and are structured with a "structuring" or "gelling" agent, conventionally a wax or a polymer, to improve the stiffness of the compositions and especially to obtain solid compositions, preferably in the form of sticks. These solid compositions remain stable and in particular do not exude, especially at room temperature.

Needless to say, the galenical form of these compositions must, on the one hand, satisfy mechanical requirements in order to ensure the glidance and wear properties of the stick during application and to prevent it from breaking, and, on the other hand, satisfy transfer qualities so as to ensure comfortable application and also a sufficient and good-quality deposit on the lips.

FR2968938 discloses a solid anhydrous cosmetic composition comprising hollow particles, a volatile oil, at least one organopolysiloxane elastomer, and a wax.

FR 2959413 discloses a solid composition comprising wax, pasty compounds and silica. Besides, it is known to use raw materials such as "nanosilicas" in skin or lip products.

The use of nanosilicas allows, besides generally the obtaining of optimized properties of application such as the disintegration under the influence of the cutting engendered by the application, which allows to deposit the product in a homogeneous way on lips, then restructuring of the deposit after application, allowing a satisfactory dressing of the cosmetic result, and/or allowing to avoid or to limit the unsightly migration of the product in the fine wrinkles of the lip contour. So, the classic compositions of make-up, and in particular glossy lipstick include classically between 2 % and 7 % in weight of nanosilicas (often treated(handled) hydrophobic, to thicken effectively oil.

However, as soon as we try to free(frank) itself from the presence of "nanosilicas", it is very complicated to obtain a compromise in terms of gelification of the oil: indeed, a unsufficiently thickened gel composition will not present a property of depositing pigments, and will have a strong trend to migrate in the fine wrinkles of the lip. On the contrary, a too much thickened and/or made gel composition will not possess good cosmetic properties, in particular to the application (it will be difficult to give evidence in a homogeneous way on lips) and will present a low (weak) shine, because of the low (weak) availability of oil, in particular not volatile oil.

We so look for an alternative means in "nanosilicas" used until now to obtain a composition of make-up and/or care, in particular make-up, of which the oil is enough to make the composition thickened, not to present the inconveniences mentioned previously, in particular which is stable and presents good properties of spreading and among which the deposit on the skin and/or the lips, in particular on lips, is brilliant and/or not migrant.

Rather, we also try to obtain compositions among which the deposit on the skin and/or the lips does not present sticky character. In a favorite way, we also try to obtain a composition among which the deposit on the skin and/or the lips presents a good level of dressing of the shine and/or dressing of the color.

In a surprising way, the inventors observed that the use of an association of hydrophobic silica aerogel particles, and at least at least one wax with a melting point greater than or equal to 60 °C, with structuring agent different from the wax allows to obtain solid and anhydrous cosmetic compositions, which are stable, which present good properties of application and the deposit of which is endowed with a satisfactory shine, is comfortable (no oily sensation, pasty and/or dry), little or not migrant and/or is little sticky.

DISCLOSURE OF INVENTION

The invention is thus relating to a solid, anhydrous composition comprising, in a physically acceptable medium:

a) wax containing at least one wax with a melting point greater than or equal to 60 °C;
b) at least one hydrophobic silica aerogel particles having a specific surface area per unit of mass (SM) ranging from 500 to 1500 $m^2$/g and a size expressed as the volume-average diameter (D[0.5]) ranging from 1 to 1500 $\mu$m; and
c) at least one structuring agent different from the wax a), which is a pasty compound chosen from petrolatum, bis-diglyceryl polyacyladipate-2 and mixtures thereof.

Another object of the invention is relating to a method of preparation of the said composition, comprising the steps of:

mixing wax containing at least one wax with a melting point greater than or equal to 60 °C, at least one hydrophobic silica aerogel particles, and at least one structuring agent different from the wax under 50-110°C, preferably under 70-100 containing at least one wax with a melting point greater than or equal to 60 °C;

stiring the mixture until homogenization.

Another object of the invention is relating to a method for treating the keratin materials, preferably the skin and/or the lip, comprising the step of applying to the keratin material the composition

DETAILED DESCRIPTION OF THE INVENTION

The term "solid" composition means a composition which hardness at 20°C and at atmospheric pressure (760 mmHg) is greater than or equal to 30 Nm$^{-1}$ when it is measured according to the protocol described below.
The hardness of a solid composition is measured according to the following protocol.
The composition whose hardness is to be determined is stored at 20°C for 24 hours before measuring the hardness.
The hardness may be measured at 20°C via the "cheese wire" method, which consists in transversely cutting a wand of product, which is preferably a circular cylinder, by means of a rigid tungsten wire 250 $\mu$m in diameter, by moving the wire relative to the stick at a speed of 100 mm/minute.
The hardness of the samples of compositions of the invention, expressed in Nm$^{-1}$, is measured using a DFGS2 tensile testing machine from the company Indelco-Chatillon.
The measurement is repeated three times and then averaged. The average of the three values read using the tensile testing machine mentioned above, noted Y, is given in grams. This average is converted into Newtons and then divided by L which represents the longest distance through which the wire passes. In the case of a cylindrical wand, L is equal to the diameter (in metres).
The hardness is converted into Nm$^{-1}$ by the equation below:

$$(Y \times 10\text{-}3 \times 9.8)/L$$

For a measurement at a different temperature, the stick is stored for 24 hours at this new temperature before the measurement.
According to this measuring method, the composition according to the invention preferably has a hardness at 20°C and at atmospheric pressure of greater than or equal to 40 Nm$^{-1}$ and preferably greater than 50 Nm$^{-1}$.
Preferably, the composition according to the invention especially has a hardness at 20°C of less than 500 Nm$^{-1}$, especially less than 400 Nm$^{-1}$ and preferably less than 300 Nm$^{-1}$. Advantageously, these compositions have a shear value ranging from 50 to 120 and preferably from 70 to 100 gF. Thus, these compositions may be formulated in standard packaging that does not require any composition support means. The composition according to the invention is solid.
The composition according to the invention is anhydrous.
For the purposes of the invention, the term "anhydrous composition" means a composition containing less than 2% and preferably less than 0.5% by weight of water relative to the total weight of the composition. Where appropriate, such small amounts of water may be provided by ingredients of the composition that contain it in residual amount, but are not deliberately provided.
Preferably, the "keratin material" according to the present invention is the skin and the lips. By "skin", we intend all the body skin, including the scalp. Still preferably, the keratin material is the lips.

DETAILED DESCRIPTION OF THE INVENTION

**Physiologically acceptable medium**

A composition according to the invention comprises a physiologically acceptable medium.
The term "physiologically acceptable medium" is intended to denote a medium that is particularly suitable for applying a composition according to the invention to the skin or the lips. The physiologically acceptable medium is generally adapted to the nature of the support onto which the composition is to be applied, and also to the form in which the composition is to be packaged.

**Waxes**

According to the present invention, the composition comprises waxes. The waxes contain at least one wax with a melting point greater than or equal to 60 °C.

The wax under consideration in the context of the present invention is generally a lipophilic compound that is solid at room temperature (25°C), with a solid/liquid reversible change of state, having a melting point of greater than or equal to 60°C, which may be up to 200°C and in particular up to 120°C.

As illustrations of waxes that are suitable for the invention, mention may be made especially of hydrocarbon-based waxes, for instance beeswax, lanolin wax, Chinese insect waxes, rice bran wax, carnauba wax, candelilla wax, ouricury wax, esparto grass wax, berry wax, shellac wax, Japan wax and sumach wax; montan wax, orange wax and lemon wax, microcrystalline waxes, paraffins and ozokerite; polyethylene waxes, the waxes obtained by Fischer-Tropsch synthesis and waxy copolymers, and also esters thereof, fatty acids or esters obtained by catalytic hydrogenation of animal or plant oils containing linear or branched $C_8$-$C_{32}$ fatty chains, preferably $C_{16}$ to $C_{18}$ chains, silicone waxes and fluoro waxes, or a mixture thereof.

Mentions may be made of polyethylene which, for example, is sold under the tradename Performalene 500-L Polyethylene by the company New Phase Technologies.

Mention may also be made of fatty acids obtained by catalytic hydrogenation of animal or plant oils containing linear or branched $C_8$-$C_{32}$ fatty chains, preferably $C_{16}$ to $C_{18}$ chains. Among these compounds, mention may be made especially of stearic acid, palmitic acid, or a mixture thereof. The fatty acids used in the current invention are commercially available under the trade names, for example, AEC Stearic Acid sold by A & E Connock (Perfumery & Cosmetics) Ltd., Emersol sold by Emery Oleochemical LLC, Palmitic Acid PC sold by Protameen Chemicals, Inc.

Mention may also be made of silicone waxes such as $C_{30\text{-}45}$ alkyl dimethicone; and fluoro waxes.

As for esters obtained by catalytic hydrogenation of animal or plant oils, mention may be made to the waxes obtained by hydrogenation of castor oil esterified with cetyl alcohol, sold under the names Phytowax ricin 16L64® and 22L73® by the company Sophim, may also be used. Such waxes are described in patent application FR-A-2 792 190.

The waxes obtained by hydrogenation of olive oil esterified with $C_{12}$ to $C_{18}$ chain fatty alcohols such as those sold by the company SOPHIM under the brand names Phytowax Olive 12L44, 14L48, 16L55 and 18L57, are also convenient.

A wax that may be used is a $C_{20}$-$C_{40}$ alkyl (hydroxystearyloxy)stearate (the alkyl group containing from 20 to 40 carbon atoms), alone or as a mixture. Such a wax is especially sold under the names Kester Wax K 82 P®, Hydroxypolyester K 82 P® and Kester Wax K 80 P® by the company Koster Keunen.

Preferably, the composition according to the invention comprises waxes chosen from polyethylene, hydrogenated esters obtained by catalytic hydrogenation of plant oils containing linear or branched $C_8$-$C_{32}$ fatty chains, and more particularly hydrogenated myristyl olive ester, and a mixture thereof.

Advantageously, a composition of the invention may comprise from 1% to 20% by weight and preferably from 2% to 15% by weight and more preferably from 5% to 10% by weight of wax(es) relative to the total weight of the said composition.

**Hydrophobic silica aerogels:**

The composition of the present invention comprises at least one hydrophobic silica aerogel particles.

Silica aerogels are porous materials obtained by replacing (by drying) the liquid component of a silica gel with air. They are generally synthesized via a sol-gel process in liquid medium and then dried, usually by extraction of a supercritical fluid; the one most commonly used being supercritical $CO_2$. This type of drying makes it possible to avoid shrinkage of the pores and of the material. The sol-gel process and the various drying processes are described in detail in Brinker CJ., and Scherer G.W., Sol-Gel Science: New York: Academic Press, 1990.

The hydrophobic silica aerogel particles used in the present invention have a specific surface area per unit of mass (SM) ranging from 500 to 1500 m2/g, preferably from 600 to 1200 m2/g and better still from 600 to 800 m2/g, and a size expressed as the mean volume diameter (D[0.5]), ranging from 1 to 30 $\mu$m, preferably from 5 to 25 $\mu$m, better still from 5 to 20 $\mu$m and even better still from 5 to 15 $\mu$m.

The specific surface area per unit of mass may be determined via the BET (Brunauer-Emmett-Teller) nitrogen absorption method described in the Journal of the American Chemical Society, vol. 60, page 309, February 1938 and corresponding to the international standard ISO 5794/1 (appendix D). The BET specific surface area corresponds to the total specific surface area of the particles under consideration.

The size of the silica aerogel particles may be measured by static light scattering using a commercial granulometer such as the MasterSizer 2000 machine from Malvern. The data are processed on the basis of the Mie scattering theory. This theory, which is exact for isotropic particles, makes it possible to determine, in the case of non-spherical particles, an "effective" particle diameter. This theory is especially described in the publication by Van de Hulst, H.C.,

"Light Scattering by Small Particles," Chapters 9 and 10, Wiley, New York, 1957.

According to one advantageous embodiment, the hydrophobic silica aerogel particles used in the present invention have a specific surface area per unit of mass (SM) ranging from 600 to 800 m2/g and a size expressed as the mean volume diameter (D[0.5]) ranging from 5 to 20 μm and better still from 5 to 15 μm.

The silica aerogel particles used in the present invention may advantageously have a tamped density r) ranging from 0.04 g/cm3 to 0.10 g/cm3 and preferably from 0.05 g/cm3 to 0.08 g/cm3.

In the context of the present invention, this density, known as the tamped density, may be assessed according to the following protocol:

40 g of powder are poured into a measuring cylinder; the measuring cylinder is then placed on a Stav 2003 machine from Stampf Volumeter; the measuring cylinder is then subjected to a series of 2500 packing motions (this operation is repeated until the difference in volume between two consecutive tests is less than 2%); the final volume Vf of packed powder is then measured directly on the measuring cylinder. The tamped density is determined by the ratio m/Vf, in this instance 40/Vf (Vf being expressed in cm3 and m in g).

According to one embodiment, the hydrophobic silica aerogel particles used in the present invention have a specific surface area per unit of volume SV ranging from 5 to 60 m2/cm3, preferably from 10 to 50 m2/cm3 and better still from 15 to 40 m2/cm3.

The specific surface area per unit of volume is given by the relationship:

SV = SM.r where r is the tamped density expressed in g/cm3 and SM is the specific surface area per unit of mass expressed in m2/g, as defined above.

Preferably, the hydrophobic silica aerogel particles according to the invention have an oil-absorbing capacity, measured at the wet point, ranging from 5 to 18 ml/g, preferably from 6 to 15 ml/g and better still from 8 to 12 ml/g.

The oil-absorbing capacity measured at the wet point, noted Wp, corresponds to the amount of water that needs to be added to 100 g of particle in order to obtain a homogeneous paste.

It is measured according to the wet point method or the method for determining the oil uptake of a powder described in standard NF T 30-022. It corresponds to the amount of oil adsorbed onto the available surface of the powder and/or absorbed by the powder by measuring the wet point, described below:

An amount = 2 g of powder is placed on a glass plate, and the oil (isononyl isononanoate) is then added dropwise. After addition of 4 to 5 drops of oil to the powder, mixing is performed using a spatula, and addition of oil is continued until a conglomerate of oil and powder has formed. At this point, the oil is added one drop at a time and the mixture is then triturated with the spatula. The addition of oil is stopped when a firm, smooth paste is obtained. This paste must be able to be spread on the glass plate without cracking or forming lumps. The volume Vs (expressed in ml) of oil used is then noted. The oil uptake corresponds to the ratio Vs/m.

The aerogels used according to the present invention are hydrophobic silica aerogels, preferably of silylated silica (INCI name: silica silylate).

The term "hydrophobic silica" means any silica whose surface is treated with silylating agents, for example halogenated silanes such as alkylchlorosilanes, siloxanes, in particular dimethylsiloxanes such as hexamethyldisiloxane, or silazanes, so as to functionalize the OH groups with silyl groups Si-Rn, for example trimethylsilyl groups.

As regards the preparation of hydrophobic silica aerogels particles that have been surface-modified by silylation, reference may be made to document US 7 470 725.

Use will be made in particular of hydrophobic silica aerogels particles surface-modified with trimethylsilyl groups .

As hydrophobic silica aerogels that may be used in the invention, examples that may be mentioned include the aerogel sold under the name VM-2260 (INCI name: Silica silylate), by the company Dow Corning, the particles of which have a mean size of about 1000 microns and a specific surface area per unit of mass ranging from 600 to 800 m2/g.

Mention may also be made of the aerogels sold by the company Cabot under the references Aerogel TLD 201, Aerogel OGD 201 and Aerogel TLD 203, Enova Aerogel MT 1100 and Enova Aerogel MT 1200.

Use will be made more particularly of the aerogel sold under the name VM-2270 (INCI name: Silica silylate), by the company Dow Corning, the particles of which have a mean size ranging from 5-15 microns and a specific surface area per unit of mass ranging from 600 to 800 m2/g. The silica aerogel particles in accordance with the invention are preferably present in the cosmetic composition in an amount of active material ranging from 0.1% to 10% by weight and more preferentially from 0.3% to 5% by weight, and more preferably from 0./8% to 2% by weight of the silica aerogel particles relative to the total weight of the composition.

**Structuring agent**

The composition comprises, besides the waxes having a melting point higher or equal to 60°C present in the composition, at least one structuring agent different from the said wax(es), which is a pasty compound chosen from petrolatum, bis-diglyceryl polyacyladipate-2 and mixtures thereof.

The term "pasty compounds" within the meaning of the present invention is understood to mean a lipophilic fatty compound with a reversible solid/liquid change in state which exhibits, in the solid state, an anisotropic crystalline arrangement and which comprises, at a temperature of 23°C, a liquid fraction and a solid fraction.

In other words, the starting melting point of the pasty fatty substance can be less than 23°C. The liquid fraction of the pasty compounds, measured at 23°C, can represent from 9 to 97% by weight of the pasty compounds. At 23°C, this liquid fraction preferably represents between 15 and 85% by weight, more preferably between 40 and 85% by weight.

Within the meaning of the invention, the melting point corresponds to the temperature of the most endothermic peak observed by thermal analysis (DSC) as described in Standard ISO 11357-3: 1999. The melting point of a pasty compounds can be measured using a differential scanning calorimeter (DSC), for example the calorimeter sold under the name "MDSC 2920" by TA Instruments.

The measurement protocol is as follows:

A 5 mg sample of pasty compounds placed in a crucible is subjected to a first rise in temperature ranging from -20°C to 100°C at a heating rate of 10°C/minute, is then cooled from 100°C to -20°C at a cooling rate of 10°C/minute and, finally, is subjected to a second rise in temperature ranging from -20°C to 100°C at a heating rate of 5°C/minute. During the second rise in temperature, the variation in the difference in power absorbed by the empty crucible and by the crucible comprising the sample of pasty compounds is measured as a function of the temperature. The melting point of the pasty compounds is the value of the temperature corresponding to the tip of the peak of the curve representing the variation in the difference in power absorbed as a function of the temperature.

The liquid fraction by weight of the pasty compounds at 23°C is equal to the ratio of the enthalpy of fusion consumed at 23°C to the enthalpy of fusion of the pasty fatty substance.

The enthalpy of fusion of the pasty fatty substance is the enthalpy consumed by the latter to change from the solid state to the liquid state. The pasty fatty substance is "in the solid state" when the whole of its mass is in the solid crystalline form. The pasty compounds is "in the liquid state" when the whole of its mass is in the liquid form.

The enthalpy of fusion of the pasty compounds is equal to the area under the curve of the thermogram obtained using a differential scanning calorimeter (DSC), such as the calorimeter sold under the name MDSC 2920 by TA Instruments, with a rise in temperature of 5 or 10°C per minute, according to Standard ISO 11357-3:1999.

The enthalpy of fusion of the pasty compounds is the amount of energy necessary to change the pasty compounds from the solid state to the liquid state. It is expressed in J/g.

The enthalpy of fusion consumed at 23°C is the amount of energy absorbed by the sample to change from the solid state to the state which it exhibits at 23°C, composed of a liquid fraction and of a solid fraction.

The liquid fraction of the pasty compounds measured at 32°C preferably represents from 30 to 100% by weight of the pasty compounds, preferably from 50 to 100% by weight of the pasty compounds, more preferably from 60 to 100% by weight of the pasty compounds. When the liquid fraction of the pasty compounds measured at 32°C is equal to 100%, the temperature of the end of the melting range of the pasty compounds is less than or equal to 32°C.

The liquid fraction of the pasty compounds measured at 32°C is equal to the ratio of the enthalpy of fusion consumed at 32°C to the enthalpy of fusion of the pasty compounds. The enthalpy of fusion consumed at 32°C is calculated in the same way as the enthalpy of fusion consumed at 23°C.

The pasty compound is chosen from

- the product whose INCI name is petrolatum and which is sold under the name Ultima White PET USP by the company Calumet Specialty,
- esters chosen from:

  • esters of a glycerol oligomer, especially diglycerol esters, in particular condensates of adipic acid and of glycerol, for which some of the hydroxyl groups of the glycerols have reacted with a mixture of fatty acids such as stearic acid, capric acid, stearic acid and isostearic acid, and 12-hydroxystearic acid, preferably such as bis-diglyceryl polyacyladipate-2 sold under the brand name Softisan 649 by the company Cremer Oleo and mixtures thereof.

**Nonvolatile oil**

According to a particular embodiment of the invention, the composition may further comprise at least one nonvolatile oil.

The term "nonvolatile oil" means an oil that remains on keratin materials, at room temperature and atmospheric pressure, for at least several hours and that especially has a vapor pressure of less than $10^{-3}$ mmHg (0.13 Pa). A nonvolatile oil may also be defined as having an evaporation rate such that, under the conditions defined previously, the amount evaporated after 30 minutes is less than 0.07 mg/cm$^2$.

The oil may be chosen from polar oils, apolar oils, or mixtures thereof.

Polar oils

The composition according to the invention may comprise at least one polar nonvolatile oil, preferably chosen from hydrocarbon-based oils and fluoro oils.

According to one preferred mode, the composition does not comprise any nonvolatile silicone oil(s).

The term "silicone oil" means an oil containing at least one silicon atom, and especially containing Si-O groups.

The term "fluoro oil" means an oil containing at least one fluorine atom.

According to a first preferred embodiment, said nonvolatile polar oil is a hydrocarbon-based oil. For the purposes of the present invention, the term "polar oil" means an oil whose solubility parameter at 25°C, $\delta a$, is other than 0 (J/cm3)1/2.

These oils may be of vegetable, mineral or synthetic origin.

The term "hydrocarbon-based oil" means an oil formed essentially from, or even constituted by, carbon and hydrogen atoms, and optionally oxygen and nitrogen atoms, and not containing any silicon or fluorine atoms. It may contain alcohol, ester, ether, carboxylic acid, amine and/or amide groups.

Nonvolatile hydrocarbon-based oil

In particular, the nonvolatile polar hydrocarbon oil may be chosen from the list of oils below, and mixtures thereof:

- hydrocarbon-based plant oils such as liquid triglycerides of fatty acids containing from 4 to 10 carbon atoms, for instance heptanoic or octanoic acid triglycerides, jojoba oil, sesame oil (820.6 g/mol);

- hydrocarbon-based mono- or di- esters the carboxylic acid residue contains from 2 to 30 carbon atoms, and the alcohol residue represents a hydrocarbon-based chain containing from 1 to 30 carbon atoms, such as isononyl isononanoate, isotridecyl isononanoate, diisostearyl malate, oleyl erucate or 2-octyldodecyl neopentanoate; isopropyl myristate;

- polyesters obtained by condensation of an unsaturated fatty acid dimer and/or trimer and of diol, such as those described in patent application FR 0 853 634, in particular such as dilinoleic acid and 1,4-butanediol. Mention may especially be made in this respect of the polymer sold by Biosynthis under the name Viscoplast 14436H (INCI name: Dilinoleic Acid/Butanediol Copolymer), or copolymers of polyols and of diacid dimers, and esters thereof, such as Hailuscent ISDA,

- fatty alcohols containing from 12 to 26 carbon atoms, saturated or unsaturated, which are branched or linear, for instance octyldodecanol, 2-butyloctanol, 2-hexyldecanol, 2-undecylpentadecanol, oleyl alcohol or isostearyl alcohol; Preferably, the said alcohols are branched; mentions maybe made of octyldodecanol such as the product with the trade name Eutanol G® sold by the company BASF;

- saturated or unsaturated $C_{12}$-$C_{26}$ fatty acids, such as oleic acid, linoleic acid and linolenic acid, and mixtures thereof;

- dialkyl carbonates, the two alkyl chains possibly being identical or different, such as dicaprylyl carbonate sold under the name Cetiol CC® by Cognis; and

- nonvolatile oils of high molecular mass, for example between 650 and 10 000 g/mol, for instance:

    i) vinylpyrrolidone copolymers such as the vinylpyrrolidone/1-hexadecene copolymer, Antaron V-216 sold or manufactured by the company ISP (MW = 7300 g/mol),
    ii) ester oils such as:

        a) linear fatty acid esters with a total carbon number ranging from 35 to 70, for instance pentaerythrityl tetrapelargonate (MW = 697.05 g/mol),
        b) hydroxylated esters such as polyglycerol-2 triisostearate (MW = 965.58 g/mol);
        c) aromatic esters such as tridecyl trimellitate (MW = 757.19 g/mol),
        d) esters of $C_{24}$-$C_{28}$ branched fatty acids or fatty alcohols such as those described in patent application EP-A-0 955 039, and especially triisoarachidyl citrate (MW = 1033.76 g/mol), pentaerythrityl tetraisononanoate (MW = 697.05 g/mol), glyceryl triisostearate (MW = 891.51 g/mol), glyceryl tris(2-decyl)tetradecanoate (MW = 1143.98 g/mol), pentaerythrityl tetraisostearate (MW = 1202.02 g/mol), polyglyceryl-2 tetraisostearate (MW = 1232.04 g/mol) or else pentaerythrityl tetrakis(2-decyl)tetradecanoate (MW = 1538.66 g/mol),
        e) esters and polyesters of a diol dimer and of a monocarboxylic or dicarboxylic acid, such as esters of a diol dimer and of a fatty acid and esters of a diol dimer and of a dicarboxylic acid dimer; mention may be made especially of the esters of dilinoleic diacids and of dilinoleyl diol dimers sold by the company Nippon Fine Chemical under the trade names Lusplan DD-DA5® and DD-DA7®,

- and mixtures thereof.

The esters of a diol dimer and of a monocarboxylic acid may be obtained from a monocarboxylic acid containing from 4 to 34 carbon atoms and especially from 10 to 32 carbon atoms, which acids are linear or branched, and saturated or unsaturated.

As illustrative examples of monocarboxylic acids that are suitable for use in the invention, mention may be made especially of fatty acids.

The esters of diol dimer and of dicarboxylic acid may be obtained from a dicarboxylic acid dimer derived in particular from the dimerization of an unsaturated fatty acid especially of $C_8$ to $C_{34}$, especially $C_{12}$ to $C_{22}$, in particular $C_{16}$ to $C_{20}$ and more particularly $C_{18}$.

According to one particular variant, it is more particularly the dicarboxylic acid dimer from which the diol dimer to be esterified is also derived.

The esters of a diol dimer and of a carboxylic acid may be obtained from a diol dimer produced by catalytic hydrogenation of a dicarboxylic acid dimer as described previously, for example hydrogenated dilinoleic diacid.

Preferably, the composition according to the invention comprises at least one polar nonvolatile oil chosen from hydrocarbon-based polar oils, preferably chosen from ester oils and alcohol oils, and mixtures thereof.

Preferably, the polar nonvolatile oil is diisostearyl malate (for instance the compound sold by the company Lubrizol under the trade name Schercemol™ Dism Ester), octyldodecanol (for instance Eutanol® G from the ABSF company), and a mixture thereof.

Preferably, the hydrocarbon-based nonvolatile polar oil is present in a total content of between 1% and 80%.

Preferably, the hydrocarbon-based nonvolatile polar oil is present in a total content of between 3% and 70% by weight, preferably between 5% and 60%by weight relative to the weight of the composition.

Nonvolatile fluoro oil

The nonvolatile oil may also be a fluoro oil.

The term "fluoro oil" means an oil comprising at least one fluorine atom.

The fluoro oils that may be used according to the invention may be chosen from fluorosilicone oils, fluoro polyethers and fluorosilicones as described in document EP-A-847 752, and perfluoro compounds.

According to the invention, the term "perfluoro compounds" means compounds in which all the hydrogen atoms have been replaced with fluorine atoms.

According to one particularly preferred embodiment, the fluoro oil according to the invention is chosen from perfluoro oils.

As examples of perfluoro oils that may be used in the invention, mention may be made of perfluorodecalins and perfluoroperhydrophenanthrenes.

According to one particularly preferred embodiment, the fluoro oil is chosen from perfluoroperhydrophenanthrenes, and especially the Fiflow® products sold by the company Creations Couleurs. In particular, use may be made of the fluoro oil for which the INCI name is Perfluoroperhydrophenanthrene, sold under the reference Fiflow 220 by the company F2 Chemicals.

Hydrocarbon-based apolar oils

The composition according to the invention preferably comprise, a hydrocarbon-based apolar non-volatile oil.

According to an embodiment, the composition advantageously comprises less than 35%, preferably less than 22% and more preferably less than 20% by weight of apolar nonvolatile oil(s), which are preferably hydrocarbon-based, relative to the total weight of the composition.

If present, the amount of apolar non volatile oil(s) in the composition represents from 1% to 34.5% by weight, relative to the total weight of the composition. Preferably, the amount of apolar non volatile oil is of at least 5% by weight, relative to the total weight of the composition ??

For the purposes of the present invention, the term "apolar oil" means an oil whose solubility parameter at 25°C, $\delta a$, is equal to 0 (J/cm3)1/2.

The definition and calculation of the solubility parameters in the Hansen three-dimensional solubility space are described in the article by C.M. Hansen: "The three dimensional solubility parameters", J. Paint Technol. 39, 105 (1967).

According to this Hansen space:

- δD characterizes the London dispersion forces derived from the formation of dipoles induced during molecular impacts;

- δp characterizes the Debye interaction forces between permanent dipoles and also the Keesom interaction forces between induced dipoles and permanent dipoles;

- δh characterizes the specific interaction forces (such as hydrogen bonding, acid/base, donor/acceptor, etc.); and

- δa is determined by the equation: $\delta a = (\delta p^2 + \delta h^2)^{1/2}$.

The parameters δp, δh, δD and δa are expressed in $(J/cm3)^{1/2}$.

The term "hydrocarbon-based oil" means an oil formed essentially from, or even constituted by, carbon and hydrogen atoms, and optionally oxygen and nitrogen atoms, and not containing any silicon or fluorine atoms.

As the the nonvolatile apolar hydrocarbon-based oil is apolar, the compoundis then free of oxygen, nitrogen atom(s).

Preferably, the nonvolatile apolar hydrocarbon-based oil may be chosen from linear or branched hydrocarbons of mineral or synthetic origin, such as:

- liquid paraffin or derivatives thereof,
- squalane,
- isoeicosane,
- liquid petroleum jelly,
- naphthalene oil,
- polybutenes such as Indopol H-100 (molar mass or MW = 965 g/mol), Indopol H-300 (MW = 1340 g/mol) and Indopol H-1500 (MW = 2160g/mol) sold or manufactured by the company Ineos,
- hydrogenated polyisobutylenes such as Parleam® sold by the company Nippon Oil Fats, Panalane H-300 E sold or manufactured by the company Amoco (MW = 1340 g/mol), Viseal 20000 sold or manufactured by the company Synteal (MW = 6000 g/mol) and Rewopal PIB 1000 sold or manufactured by the company Witco (MW = 1000 g/mol),
- decene/butene copolymers, polybutene/polyisobutene copolymers, especially Indopol L-14,
- polydecenes and hydrogenated polydecenes such as: Puresyn 10 (MW = 723 g/mol) and Puresyn 150 (MW = 9200 g/mol) sold or manufactured by the company Mobil Chemicals,
- and mixtures thereof.

Preferably, if present in the composition according to the invention, the nonvolatile apolar hydrocarbon-based oil is polybutene.

**Galenic form**

The composition of the present invention is suitable to be used as a skin care, make up or cosmetic treatment product. More particularly, the composition of the present invention is in the form of make up product such as lip balm, lip stick, lip gloss, and so on.

Additives

In a particular embodiment, a cosmetic composition according to the invention further comprises at least one compound chosen from, hydrophilic solvents, lipophilic solvents and oils different from the non volatiles oils cited before, and mixtures thereof.

A cosmetic composition according to the invention may also comprise any additive usually used in the field under consideration, chosen, for example, from gums, anionic, cationic, amphoteric or nonionic surfactants, silicone surfactants, resins, thickening agents, dispersants, antioxidants, essential oils, preserving agents, fragrances, neutralizers, antiseptics, UV-screening agents, cosmetic active agents, such as vitamins, moisturizers, emollients or collagen-protecting agents, colorants, and mixtures thereof.

It is a matter of routine operations for a person skilled in the art to adjust the nature and amount of the additives present in the compositions in accordance with the invention such that the desired cosmetic properties and stability properties thereof are not thereby affected.

The composition according to the invention may be prepared in a conventional manner.

The examples that follow are given as nonlimiting illustrations of the present invention. The percentages are weight percentages.

**EXAMPLES**

Formulation examples

The following formulation examples are prepared (inv exp stands for invention example; compa exp stands for comparative example):

| INCI name | % of ingredient by raw material (wt%) | | | |
|---|---|---|---|---|
| | Inv exp 1 | Compa exp 1 | Compa exp 2 | Compa exp 3 |
| Polyethylene (Performalene 500-L from New Phase Technologies) | 5.4 | 5.4 | 5.4 | 5.8 |
| Hydrogenated myristyl olive esters (Phytowax® olive 14L48 from Sophim) | 2.8 | 2.8 | 2.8 | 3.2 |
| Silica silylate (Dow Corning VM-2270 Aerogel Fine Particles from Dow Corning) | 0.8 | 0.8 | 0 | 0 |
| Silica (Solesphere H51 from AGC SI-TECH) | 0 | 0 | 0.8 | 0 |
| Petrolatum (Ultima white pet usp from Calumet Specialty) | 15 | 0 | 15 | 15 |
| Bis-diglyceryl polyacyladipate-2 (Softisan® 649 from Cremer oleo) | 7.6 | 0 | 7.6 | 7.6 |
| Diisostearyl malate (Schercemol™ DISM ester from Lubrizol) | 17.5 | 17.5 | 17.5 | 17.5 |
| Polybutene (Indopol® H 1500 from Ineos) | 12 | 34.6 | 12 | 12 |
| Polybutene (Indopol® H 100 from Ineos) | 14.32 | 14.32 | 14.32 | 14.32 |
| Octyldodecanol (Eutanol®G from BASF) | 16 | 16 | 16 | 16 |
| Stearyl heptanoate (Tegosoft® SH from Evonik Goldschmidt) | 3.5 | 3.5 | 3.5 | 3.5 |
| Preservatives (75205) | 0.05 | 0.05 | 0.05 | 0.05 |
| Pigments | 4.2 | 4.2 | 4.2 | 4.2 |
| Mica | 0.8 | 0.8 | 0.8 | 0.8 |
| Fragrance | 0.03 | 0.03 | 0.03 | 0.03 |

Protocol of preparation

The above listed formulations examples are prepared following the steps of:

mixing all the ingredients of the invention example 1 and comparative examples 1-3 respectively, under the temperature 95°C;

stiring the mixture until homogenization.

Evaluation example

Evaluation on the stability of the shape, conditioning effect such as moisturizing, hydration effect, make up effect pay-off and spreadability effect of the invention example and comparative examples 1 to 3 are performed.
Stability of the shape is evaluated using crash test, by following steps:

heat the invention and comparative examples to 38 °C for 24 hours;

apply the example to the lips under the heated temperature.

Conditioning effect is evaluated by 6 panelists, by applying the invention example 1 and the comparative examples 1 to 3 to the lips.

Make up effect such as spreadability and pay-off properties are evaluated by 5 experts by the following steps:

repeatly apply the product 3 times on the same area of the forearm using the same force; weigh the weight loss of the product;

measure the size of the area on the forearm where the product is applied;

calculate the weight loss per square centimeter.

Finally, comments or scores are given by the experts on the above mentioned properties.

5: very good;

4: basically good;

3: acceptable;

2: slightly poor and not acceptable;

1: poor, not acceptable.

| Properties | Scores of the examples | | | |
|---|---|---|---|---|
| | Inv exp 1 | Compa exp 1 | Compa exp 2 | Compa exp 3 |
| Stability of the shape | Stable over 10 stroke | Broken after 3 stroke | Stable over 10 stroke | Stable over 10 stroke |
| Spreadability | 5 | 2 | 5 | 5 |
| Pay-off score (weight by mg/cm2) | 5 (1.13) | 1 (0.55) | 2 (0.83) | 2 (0.75) |
| Conditioning effect | 5 | 2 | 3 | 2 |

Based on the above listed evaluation results, the inventors discovered that the composition according to the present invention overcomes the technical issues existing in the prior art, and provides a stable cosmetic composition with good conditioning effect and make up effect.

## Claims

1.   A solid anhydrous composition comprising, in a physically acceptable medium,

a) wax containing at least one wax with a melting point greater than or equal to 60 °C;
b) at least one hydrophobic silica aerogel particles having a specific surface area per unit of mass (SM) ranging from 500 to 1500 $m^2/g$ and a size expressed as the volume-average diameter (D[0.5]) ranging from 1 to 1500 $\mu$m; and
c) at least one structuring agent different from the wax a), which is a pasty compound chosen from petrolatum, bis-diglyceryl polyacyladipate-2 and mixtures thereof.

2.   Composition of claim 1 is solid.

3.   Composition of claim 1, wherein the wax is selected from the group consisting of hydrocarbon-based waxes such as beeswax, lanolin wax, Chinese insect waxes, rice bran wax, carnauba wax, candelilla wax, ouricury wax, esparto grass wax, berry wax, shellac wax, Japan wax and sumach wax; montan wax, orange wax and lemon wax, microcrystalline waxes, paraffins and ozokerite; polyethylene waxes, the waxes obtained by Fischer-Tropsch synthesis and waxy copolymers, and also esters thereof; fatty acids obtained by catalytic hydrogenation of animal or plant oils containing linear or branched $C_8$-$C_{32}$ fatty chains, preferably $C_{16}$ to $C_{18}$ chains, silicone waxes and fluoro waxes, or a mixture thereof.

4. Composition of claim 1 or 3, wherein the wax is chosen from polyethylene, hydrogenated myristyl olive ester, or a mixture thereof.

5. Composition of claim 1, wherein the wax is present in the composition from 1% to 20%, preferably from 2% to 15%, more preferably from 5% to 10% by weight relative to the weight of the composition.

6. The composition of any one of the preceding claims, wherein the hydrophobic silica aerogel particles have a specific surface area per unit of mass ranging from 600 to 1200 $m^2$/g and better still from 600 to 800 $m^2$/g.

7. The composition of any one of the preceding claims, wherein the hydrophobic silica aerogel particles have a size expressed as the volume-average diameter ranging from 1 to 1000 $\mu$m, preferably from 1 to 100 $\mu$m, in particular from 1 to 30 $\mu$m, more preferably from 5 to 25 $\mu$m, better still from 5 to 20 $\mu$m and even better still from 5 to 15 $\mu$m.

8. The composition of any one of the preceding claims, wherein the hydrophobic silica aerogel particles have a specific surface area per unit of mass (SM) ranging from 600 to 800 $m^2$/g and a size expressed as the volume-average diameter (D[0.5]) ranging from 5 to 20 $\mu$m and better still from 5 to 15 $\mu$m.

9. The composition of any one of the preceding claims, wherein the hydrophobic silica aerogel particles have a tapped density ranging from 0.04 $g/cm^3$ to 0.10 $g/cm^3$ and preferably from 0.05 $g/cm^3$ to 0.08 $g/cm^3$.

10. The composition of any one of the preceding claims, wherein the hydrophobic silica aerogel particles have a specific surface area per unit of volume SV ranging from 5 to 60 $m^2/cm^3$, preferably from 10 to 50 $m^2/cm^3$ and better still from 15 to 40 $m^2/cm^3$.

11. The composition of any one of the preceding claims, wherein the hydrophobic silica aerogel particles have an oil absorption capacity, measured at the wet point, ranging from 5 to 18 ml/g, preferably from 6 to 15 ml/g and better still from 8 to 12 ml/g of particles.

12. The composition of any one of the preceding claims, wherein the hydrophobic silica aerogel particles are surface-modified with trimethylsilyl groups.

13. The composition according to any one of the preceding claims, wherein the composition comprises from 0.1% to 10% by weight and more preferentially from 0.3% to 5% by weight, and more preferably from 0.8% to 2% by weight of the silica aerogel particles relative to the total weight of the composition.

14. Composition of claim 1, wherein the structuring agent is present in the composition from 1% to 50%, preferably from 5% to 30%, more preferably from 10% to 25% by weight relative to the weight of the composition.

15. Composition of any one of preceding claims, further comprising at least one non-volatile oil, preferably chosen from polybutene, diisostearyl malate, octyldodecanol, and a mixture thereof.

**Patentansprüche**

1. Feste wasserfreie Zusammensetzung, die in einem physikalisch akzeptablen Medium Folgendes umfasst:

   a) Wachs, das mindestens ein Wachs mit einem Schmelzpunkt größer oder gleich 60°C enthält;
   b) Partikel aus mindestens einem hydrophoben Silicaaerogel mit einer spezifischen Oberfläche pro Masseeinheit (SM) im Bereich von 500 bis 1500 $m^2$/g und einer als volumengemittelter Durchmesser (D[0,5]) ausgedrückten Größe im Bereich von 1 bis 1500 $\mu$m; und
   c) mindestens ein von dem Wachs a) verschiedenes Strukturierungsmittel, bei dem es sich um eine pastöse Masse handelt, die aus Petrolatum, bis-Diglyceryl, Polyacyladipat-2 und Mischungen davon ausgewählt ist.

2. Zusammensetzung nach Anspruch 1, die ein Feststoff ist.

3. Zusammensetzung nach Anspruch 1, wobei das Wachs aus der Gruppe ausgewählt ist, die aus kohlenwasserstoffbasierten Wachsen besteht wie zum Beispiel Bienenwachs, Wollwachs, chinesisches Insektenwachs, Reiskleiewachs, Karnaubawachs, Candelillawachs, Ouricurywachs, Espartograswachs, Beerenwachs, Schellackwachs, Japanwachs und Sumachwachs; Montanwachs, Orangenwachs und Zitronenwachs, mikrokristalline Wachse, Paraffine und Ozokerit; Polyethylenwachse, die durch Fischer-Tropsch-Synthese gewonnenen Wachse und wachsartige Copolymere und auch deren Ester; durch katalytische Hydrierung von tierischen oder pflanzlichen Ölen gewonnene Fettsäuren, die lineare oder verzweigte $C_8$-$C_{32}$-Fettketten, vorzugsweise $C_{16}$-$C_{18}$-Ketten enthalten, Siliconwachse und Fluorwachse oder eine Mischung davon.

4. Zusammensetzung nach Anspruch 1 oder 3, wobei das Wachs aus Polyethylen, hydriertem Myristylolivenester oder einer Mischung davon ausgewählt ist.

5. Zusammensetzung nach Anspruch 1, wobei das Wachs in der Zusammensetzung in einer Menge von 1 bis 20 Gew.-%, vorzugsweise von 2 bis 15 Gew.-%, mehr bevorzugt von 5 bis 10 Gew.-% bezogen auf das Gewicht der Zusammensetzung vorliegt.

6. Zusammensetzung nach einem der vorhergehenden Ansprüche, wobei die hydrophoben Silicaaerogelpartikel eine spezifische Oberfläche pro Masseeinheit im Bereich von 600 bis 1200 $m^2$/g und noch besser von 600 bis 800 $m^2$/g besitzen.

7. Zusammensetzung nach einem der vorhergehenden Ansprüche, wobei die hydrophoben Silicaaerogelpartikel eine als volumengemittelter Durchmesser ausgedrückte Größe im Bereich von 1 bis 1000 $\mu$m, vorzugsweise von 1 bis 100 $\mu$m, insbesondere von 1 bis 30 $\mu$m, mehr bevorzugt von 5 bis 25 $\mu$m, noch besser von 5 bis 20 $\mu$m und sogar noch besser von 5 bis 15 $\mu$m besitzen.

8. Zusammensetzung nach einem der vorhergehenden Ansprüche, wobei die hydrophoben Silicaaerogelpartikel eine spezifische Oberfläche pro Masseeinheit (SM) im Bereich von 600 bis 800 $m^2$/g und eine als volumengemittelter Durchmesser (D[0,5]) ausgedrückte Größe im Bereich von 5 bis 20 $\mu$m und noch besser von 5 bis 15 $\mu$m besitzen.

9. Zusammensetzung nach einem der vorhergehenden Ansprüche, wobei die hydrophoben Silicaaerogelpartikel eine Klopfdichte im Bereich von 0,04 g/$cm^3$ bis 0,10 g/$cm^3$ und vorzugsweise von 0,05 g/$cm^3$ bis 0,08 g/$cm^3$ besitzen.

10. Zusammensetzung nach einem der vorhergehenden Ansprüche, wobei die hydrophoben Silicaaerogelpartikel eine spezifische Oberfläche pro Volumeneinheit (SV) im Bereich von 5 bis 60 $m^2$/$cm^3$, vorzugsweise von 10 bis 50 $m^2$/$cm^3$ und noch besser von 15 bis 40 $m^2$/$cm^3$ besitzen.

11. Zusammensetzung nach einem der vorhergehenden Ansprüche, wobei die hydrophoben Silicaaerogelpartikel ein Ölabsorptionsvermögen, gemessen an der nassen Stelle, im Bereich von 5 bis 18 ml/g, vorzugsweise von 6 bis 15 ml/g und noch besser von 8 bis 12 ml/g Partikel besitzen.

12. Zusammensetzung nach einem der vorhergehenden Ansprüche, wobei die hydrophoben Silicaaerogelpartikel mit Trimethylsilylgruppen oberflächenmodifiziert sind.

13. Zusammensetzung nach einem der vorhergehenden Ansprüche, wobei die Zusammensetzung von 0,1 bis 10 Gew.-% und mehr bevorzugt von 0,3 bis 5 Gew.-% und noch mehr bevorzugt von 0,8 bis 2 Gew.-% Silicaaerogelpartikel bezogen auf das Gesamtgewicht der Zusammensetzung umfasst.

14. Zusammensetzung nach Anspruch 1, wobei das Strukturierungsmittel in der Zusammensetzung in einer Menge von 1 bis 50 Gew.-%, vorzugsweise von 5 bis 30 Gew.-%, mehr bevorzugt von 10 bis 25 Gew.-% bezogen auf das Gewicht der Zusammensetzung vorliegt.

15. Zusammensetzung nach einem der vorhergehenden Ansprüche, ferner umfassend mindestens ein nichtflüchtiges Öl, vorzugsweise ausgewählt aus Polybuten, Diisostearylmalat, Octyldodecanol und einer Mischung davon.

**Revendications**

1. Composition anhydre solide comprenant, au sein d'un milieu physiquement admissible,

(a) de la cire contenant au moins une cire dont le point de fusion est supérieur ou égal à 60 °C,

(b) des particules d'au moins un aérogel de silice hydrophobe, présentant une surface spécifique par unité de masse (aire massique ou AM) comprise dans l'intervalle allant de 500 à 1500 $m^2/g$, et une taille, exprimée par le diamètre moyen en volume (D[0,5]), comprise dans l'intervalle allant de 1 à 1500 $\mu m$,

(c) et au moins un agent structurant, différent de la cire (a), qui est un composé pâteux choisi parmi du pétrolatum, du bis-diglyceryl polyacyladipate-2 et des mélanges de ces substances.

2. Composition conforme à la revendication 1, qui est solide.

3. Composition conforme à la revendication 1, dans laquelle la cire est choisie dans l'ensemble constitué par les substances suivantes : des cires à base d'hydrocarbures, telles que cire d'abeille, cire de lanoline, cires d'insecte de Chine, cire de son de riz, cire de carnauba, cire de candelilla, cire d'ouricury, cire d'alfa, cire de baie, cire de gomme-laque, cire du Japon et cire de baie de sumac ; de la cire de montan, de la cire d'orange et de la cire de citron, des cires microcristallines, des paraffines et de l'ozocérite ; des cires de polyéthylène, les cires obtenues par synthèse de Fischer-Tropsch et des copolymères cireux, ainsi que leurs esters ; des acides gras obtenus par hydrogénation catalytique d'huiles animales ou végétales contenant des chaînes grasses en $C_8$-$C_{32}$ linéaires ou ramifiées, et de préférence des chaînes en $C_{16}$ à $C_{18}$, des cires de silicone et des cires fluorées, ou un mélange de telles substances.

4. Composition conforme à la revendication 1 ou la revendication 3, dans laquelle la cire est choisie parmi les suivantes : polyéthylène, esters de myristyle et d'huile d'olive hydrogénée, ou un mélange de telles substances.

5. Composition conforme à la revendication 1, dans laquelle la cire se trouve en une proportion, rapportée au poids de la composition, valant de 1 % à 20 % en poids, de préférence de 2 % à 15 % en poids, et mieux encore de 5 % à 10 % en poids.

6. Composition conforme à l'une quelconque des revendications précédentes, dans laquelle les particules d'aérogel de silice hydrophobe présentent une surface spécifique par unité de masse comprise dans l'intervalle allant de 600 à 1200 $m^2/g$ et encore mieux de 600 à 800 $m^2/g$.

7. Composition conforme à l'une quelconque des revendications précédentes, dans laquelle les particules d'aérogel de silice hydrophobe présentent une taille, exprimée par le diamètre moyen en volume, comprise dans l'intervalle allant de 1 à 1000 $\mu m$, de préférence de 1 à 100 $\mu m$, en particulier de 1 à 30 $\mu m$, mieux encore de 5 à 25 $\mu m$, encore mieux de 5 à 20 $\mu m$ et au mieux de 5 à 15 $\mu m$.

8. Composition conforme à l'une quelconque des revendications précédentes, dans laquelle les particules d'aérogel de silice hydrophobe présentent une surface spécifique par unité de masse (AM) comprise dans l'intervalle allant de 600 800 $m^2/g$, et une taille, exprimée par le diamètre moyen en volume (D[0,5]), comprise dans l'intervalle allant de 5 à 20 $\mu m$ et mieux encore de 5 à 15 $\mu m$.

9. Composition conforme à l'une quelconque des revendications précédentes, dans laquelle les particules d'aérogel de silice hydrophobe présentent une masse volumique tassée comprise dans l'intervalle allant de 0,04 $g/cm^3$ à 0,10 $g/cm^3$ et de préférence de 0,05 $g/cm^3$ à 0,08 $g/cm^3$.

10. Composition conforme à l'une quelconque des revendications précédentes, dans laquelle les particules d'aérogel de silice hydrophobe présentent une surface spécifique par unité de volume AV comprise dans l'intervalle allant de 5 à 60 $m^2/cm^3$, de préférence de 10 à 50 $m^2/cm^3$ et encore mieux de 15 à 40 $m^2/cm^3$.

11. Composition conforme à l'une quelconque des revendications précédentes, dans laquelle les particules d'aérogel de silice hydrophobe présentent une capacité d'absorption d'huile, déterminée au point humide, comprise dans l'intervalle allant de 5 à 18 mL/g, de préférence de 6 à 15 mL/g et mieux encore de 8 à 12 mL/g de particules.

12. Composition conforme à l'une quelconque des revendications précédentes, dans laquelle les particules d'aérogel de silice hydrophobe ont été modifiées en surface par des groupes triméthylsilyle.

13. Composition conforme à l'une quelconque des revendications précédentes, dans laquelle les particules d'aérogel de silice se trouvent en une proportion, rapportée au poids total de la composition, valant de 0,1 % à 10 % en poids, mieux encore de 0,3 % à 5 % en poids et encore mieux de 0,8 % à 2 % en poids.

**14.** Composition conforme à la revendication 1, dans laquelle l'agent structurant se trouve en une proportion, rapportée au poids de la composition, valant de 1 % à 50 % en poids, de préférence de 5 % à 30 % en poids, et mieux encore de 10 % à 25 % en poids.

**15.** Composition conforme à l'une quelconque des revendications précédentes, qui comprend par ailleurs au moins une huile non volatile, choisie de préférence parmi du polybutène, du malate de di-isostéaryle, de l'octyldodécanol, et un mélange de telles substances.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- FR 2968938
- FR 2959413
- FR 2792190 A
- US 7470725 B

- FR 0853634
- EP 0955039 A
- EP 847752 A

### Non-patent literature cited in the description

- **BRINKER CJ. ; SCHERER G.W.** Sol-Gel Science. Academic Press, 1990
- *Journal of the American Chemical Society,* February 1938, vol. 60, 309

- **VAN DE HULST, H.C.** Light Scattering by Small Particles. Wiley, 1957
- **C.M. HANSEN.** The three dimensional solubility parameters. *J. Paint Technol.,* 1967, vol. 39, 105